(19) Europäisches Patentamt
European Patent Office
Office européen des brevets

(11) Publication number : **0 478 792 B1**

(12) # EUROPEAN PATENT SPECIFICATION

(45) Date of publication of patent specification : **27.07.94 Bulletin 94/30**

(51) Int. Cl.$^5$ : **A23L 1/29,** A61K 37/02

(21) Application number : **91906699.3**

(22) Date of filing : **03.04.91**

(86) International application number : **PCT/JP91/00442**

(87) International publication number : **WO 91/15127 17.10.91 Gazette 91/24**

(54) **HIGH-PROTEIN HIGH-VISCOSITY ALIMENTARY FOOD COMPOSITION.**

(30) Priority : **06.04.90 JP 92488/90**

(43) Date of publication of application : **08.04.92 Bulletin 92/15**

(45) Publication of the grant of the patent : **27.07.94 Bulletin 94/30**

(84) Designated Contracting States : **CH DE ES FR GB IT LI**

(56) References cited :
**FR-A- 2 247 988**
**JP-A-12 401 69**
**JP-A-61 585 60**
**JP-A-62 224 259**
**JP-A-62 232 361**

(73) Proprietor : **OTSUKA PHARMACEUTICAL CO., LTD.**
**9, Kandatsukasa-cho 2-chome**
**Chiyoda-ku Tokyo 101 (JP)**

(72) Inventor : **TAKAICHI, Akihisa**
**172-3, Aza Nakajima,**
**Takashima,**
**Naruto-cho**
**Naruto-shi, Tokushima 772 (JP)**
Inventor : **WATANABE, Yoshinari**
**5-12, Aza Dekiji,**
**Otoze,**
**Aizumicho**
**Itanogun, Tokushima 771-12 (JP)**
Inventor : **KITANO, Koji, Otsuka Seiyaku K. K. Imagireryo**
**463--10, Kagasuno,**
**Kawauchicho**
**Tokushima-shi, Tokushima 771-01 (JP)**

(74) Representative : **Hansen, Bernd, Dr. Dipl.-Chem. et al**
**Hoffmann, Eitle & Partner**
**Patent- und Rechtsanwälte,**
**Postfach 81 04 20**
**D-81904 München (DE)**

**EP 0 478 792 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### INDUSTRIAL FIELD OF UTILIZATION

The present invention relates to a nutritive food composition and more particularly to a high-protein, high-viscosity nutritive food composition which is rich in proteins of high quality.

### PRIOR ART AND ITS PROBLEMS

Recent years have witnessed an increased consciousness of health, shapeup and dieting and the consequent increase in the sporting population dedicated to jogging, cycling and mountaineering. Under the circumstances, the present invention has as an object to provide a food composition enabling an easy and expedient intake of high-quality protein necessary for energy supplementation in sport activities and an augmentation of body protein, such as the muscle, under the condition of increased protein assimilation of the body during physical exercises for body building, shapeup and other purposes. It is another object of the present invention to provide a food composition which can be utilized as a space food to be supplied in squeeze-tubes, for instance. A still another object of the invention is to provide a high-protein, high-viscosity nutritive food composition for hospitalized patients which is rich in high quality protein and lean in water and can be ingested easily and conveniently even by bed-ridden patients in postoperative convalescence or in the course of recovery from liver disease, or patients with renal failure.

The inventors of the present invention explored this field of formula feeding to satisfy the above requirements and found that a food composition having the formulation and rheological profile defined below is a very effective nutritive food meeting the above requirements and the use of this particular composition enables us to satisfy the need for intensive nutrition of patients with said diseases or for shapeup and body building of healthy persons. The present invention is predicated on the above finding.

### DISCLOSURE OF THE INVENTION

In accordance with the present invention there is provided a high-protein, high-viscosity nutritive food composition characterized in that it is composed, on a dry weight basis, 40 to 65% (% by weight; the same applies hereinafter) of protein, 5 to 25% of fat and 15 to 40% of carbohydrate and has a viscosity in the range of 500 to 3.000 mPas (500 to 3,000 cp) (at 30°C, Type B viscosimeter) and an amino acid score (on a 2~5-year-old infant basis) of not less than 80.

As used in this specification, the term "amino acid score (on a 2~5-year-old infant basis)" means the score based on the following amino acid score pattern adopted by the Joint FAO/WHO/UNU Ad Hoc Expert Committee of 1985.

| Abbreviation of amino acid | Essential amino acids per unit protein (mg/g protein*) |
|---|---|
| His | 19 |
| Ile | 28 |
| Leu | 66 |
| Lys | 58 |
| Cys | 25 |
| Tyr | 63 |
| Thr | 34 |
| Trp | 11 |
| Val | 35 |
| Total | |
| (His included) | 339 |
| (His excluded) | 320 |

The amount of protein* is calculated by the formula "Nitrogen x 6.25".

By virture of the above-defined formula, the food composition of the present invention can, on oral or enteral feeding, supply adequate calorie and nutriments according to an energy feeding, muscle building or shapeup protocol or expedite postoperative restoration of body protein.

The nutritive food composition of the present invention is now described in detail. The composition of the invention can be manufactured by the established production technology for nutritive food preparations of this kind except that it is so arranged that the final composition contains the above-specified amounts of protein, fat and carbohydrate and has a viscosity and an amino acid score in the respective ranges defined hereinbefore.

The protein mentioned above includes, among others, casein and its salts, gelatin and its salts, water-soluble gelatin (e.g. enzymatically degraded gelatin), whole milk, skim milk, soybean protein, corn gluten meal, wheat protein and so on. The fat includes, among others, soybean oil, olive oil, medium-chain triglycerides (MCT), cottonseed oil, sunflower oil, cacao butter, sesame oil, rice oil, safflower oil, peanut oil, palm oil, rapeseed oil and so on. The carbohydrate includes, among others, dextrin, sucrose, monosaccharides such as fructose, glucose, etc., sugar alcohols such as erythritol, disccharides such as malt sugar or maltose, and oligosaccharides such as fructo oligosaccharides, lactooligosaccharides, galactosyllactose, lactosucrose and so on.

The proportions of the above ingredients in the food composition of the invention are chosen from within the following ranges.

| Ingredient | Permissible range (wt.%) | Preferred range (wt.%) |
|---|---|---|
| Protein | 40-65 | 40-53 |
| Fat | 5-25 | 10-18 |
| Carbohydrate | 15-40 | 20-35 |

It should be understood that the indicated amount of protein is the amount of pure protein as determined by measuring the nitrogen content of the respective proteinous materials by Kjeldahl's method.

In the high-protein, high-viscosity nutritive food composition of the invention, there may be incorporated various additives where necessary. The additives may be these commonly used in nutritive foods of this kind. Thus, among such additives are various vitamins, minerals, synthetic flavoring substances and natural flavor concentrates, natural sweeteners (sormatin, stevia, etc.), synthetic sweeteners (saccharin, stevia extract, aspartame, etc.), colorants, flavors (cheese, chocolate, etc.), and dietary fibers such as polydextrose, pectic acid and its salts, alginic acid and its salts, etc. and these additives can be used singly or in combination. While the proportion of such additives is more or less optional, it is generally chosen from within the range of 0 to about 20 parts by weight based on 100 parts by weight of the total composition of the invention.

The composition of the present invention can be manufactured by mixing the above ingredients in any optional manner. For example, all the ingredients may be mixed together in one operation. An alternative procedure comprises adding as an auxiliary emulsifier, e.g. protein, carbohydrate, etc., and where necessary an emulsifying agent, e.g. lecithin, sugar esters, etc., to a mixture of fat-soluble materials (fats, oils and other fat-soluble components) and emulsifying the resulting mixture mechanically in the routine manner to provide a composition of the invention.

The resulting composition of the invention (the food of the invention in a liquid form) can be filled into an appropriate container and sterilized by retorting (120°C, 20 minutes) to provide a product having a sufficient shelf life. This product can be used directly or as appropriately diluted.

The food composition of the invention thus manufactured insures decomposition (digestion) and absorption at appropriate rates in the intestinal tract and is low in osmolarity. Therefore, it can exhibit its nutritional condition-improving effect effectively and in a steady manner, substantially without the risk of causing diarrhea in the recipient. The amount of the food per intake is preferably chosen from within the range of about 10 to 30 g on a dry basis or, on a bulc volume basis, about 50 to 300 cc.

The food composition of the present invention can be advantageously utilized for supplying nutrients to patients who require oral, pernasal or enteral nutrition. It is also a nourishing food (or beverage) for healthy persons. The use of this food is conductive to effective energy supplementation, muscle building, protein augmentation, shapeup, etc. and, moreover, offers the advantage that it can be simply and easily ingested.

The food according to the invention is also of use as a food for increasing body protein for purposes of muscle building, shapeup, etc. as well as a space food to be ingested from squeeze-tubes. Moreover, unlike preparations administered intravenously, e.g. by intravenous drip, the composition is physiological and can be utilized universally for all patients needing improved nutritional homeostasis.

The benefits that can be realized from the use of the food of the present invention include improvements in various parameters or indicators of nutrition, such as total protein, albumin, nitrogen balance, cholesterol, triglyceride value, triceps muscle of arm, thickness of subcutaneous fat at brachial muscle, prealbumin, retinol, transferrin, body weight, total cholesterol, triglycerides, HDL-C, apoprotein, free fatty acids and so on. Therefore, the food composition of the present invention is effective for nutritional supplementation and nutrition control in patients with hyperlipidemia or obesity. Furthermore, when it is used for improving the nutritional state of healthy persons or as a dietetic food for persons with a tendency of obesity, the desired object can be accomplished without untoward effects on their nutritional homeostasis. Thus, the composition can be used as a convenient and useful food or drink. Moreover, the food of the invention has the advantage that the risk of side effects such as diarrhea, vomiting, nausea, abdominal discomfort, etc. is minimal.

## EXAMPLES

The following preparation examples of the nutritive food composition are further illustrative of the present invention. It should be understood that all % in the examples are by weight.

Examples 1-13

Sodium caseinate, calcium caseinate, gelatin and sucrose are dissolved in water with stirring. Then, NaCl and other minerals are added and dissolved with stirring to prepare a solution A.

On the other hand, casein is dissolved in water and neutralized and dissolved by addition of NaOH. Then, $MgSO_4$ and other minerals as well as vitamins and oils are added and dissolved with stirring to prepare a solution B.

The two solutions, A and B, are blended and after stirring and volume adjustment, vitamins, flavors, etc. are added and emulsified to provide a food composition of the invention.

This liquid is filled, in 80 ml portions, into tubes and sterilized to provide a batch of products.

The ingredients, as well as their amounts, viscosity (30°C, Type B viscosimeter) and amino acid score (on a 2~5-year-old infant basis) of the above composition of the invention are presented below in Table 1.

The vitamins and minerals used and their amounts are as follows.

<Vitamins>

| | |
|---|---|
| Vitamin A | 1155 IU |
| Vitamin $B_1$ | 0.92 mg |
| Vitamin $B_2$ | 0.92 mg |
| Vitamin $B_6$ | 0.92 mg |
| Vitamin $B_{12}$ | 2.77 μg |
| Vitamin C | 34.64 mg |
| Vitamin D | 92.36 IU |
| Vitamin E | 6.93 IU |
| Pantothenic acid | 4.62 mg |
| Niacin | 9.24 mg |
| Folic acid | 184.72 μg |
| Biotin | 138.54 μg |
| Vitamin K | 69.27 μg |
| Choline | 115.45 mg |

<Minerals>

| | |
|---|---|
| Ca | 230.90 mg |
| $PO_4$ | 230.90 mg |
| Mg | 92.36 mg |
| Na | 323.26 mg |
| K | 600.34 mg |
| Cl | 461.80 mg |
| Fe | 7.39 mg |
| Zn | 3.69 mg |
| Cu | 0.46 mg |
| Mn | 9.24 mg |
| I | 34.64 μg |

Table 1

| Example No. | Example 1 | Example 2 | Example 3 |
|---|---|---|---|
| Protein (g/80 ml) | 9.5 | 10.2 | 13.0 |
| (w/w%) | 41.3 | 48.1 | 65.0 |
| Carbohydrate (g/80 ml) | 9.2 | 7.5 | 5.2 |
| (w/w%) | 40.0 | 35.4 | 26.0 |
| Fat (g/80 ml) | 4.3 | 3.5 | 1.8 |
| (w/w%) | 18.7 | 16.5 | 9.0 |
| Energy y (Kcal) | 0,477 (114) | 0,431 (103) | 0,373 (89) |
| Protein materials | | | |
| Casein | 5.0 | 4.9 | 6.9 |
| Sodium caseinate | 2.1 | - | - |
| Calcium caseinate | 2.2 | 3.7 | 3.3 |
| Whole milk | - | 4.7 | 3.9 |
| Skim milk | - | 3.0 | 1.5 |
| Gelatin | 0.8 | - | - |
| Enzymatically degraded gelatin | - | - | 2.2 |
| Wheat flour | 3.0 | - | - |
| Cheese | - | 2.7 | 2.0 |
| Carbohydrate material | | | |
| Refined sucrose | 7.0 | 2.4 | 2.7 |
| Fat materials | | | |
| Rice oil | 4.2 | 0.1 | - |
| Chocolate | - | 3.0 | - |
| Other ingredients | | | |
| Vitamins | q.s. | q.s. | q.s. |
| Minerals | q.s. | q.s. | q.s. |
| Flavors | q.s. | q.s. | q.s. |
| Viscosity, mPas (cp) (30°C) | 1780 | 840 | 2220 |
| Amino acid score | 100 | 100 | 100 |

Table 1 (continued)

| Example No. | Example 4 | Example 5 | Example 6 |
|---|---|---|---|
| Protein (g/80 ml) | 12.2 | 10.8 | 9.0 |
| (w/w%) | 61.0 | 53.2 | 45.0 |
| Carbohydrate (g/80 ml) | 3.0 | 6.9 | 8.0 |
| (w/w%) | 15.0 | 41.7 | 40.0 |
| Fat (g/80 ml) | 5.0 | 2.8 | 3.0 |
| (w/w%) | 25.0 | 14.1 | 15.0 |
| Energy y (Kcal) | 0,440 (105) | 0,394 (94) | 0,398 (95) |
| Protein materials | | | |
| Casein | 6.7 | 5.6 | 5.6 |
| Sodium caseinate | 2.2 | 1.1 | - |
| Calcium caseinate | 3.3 | 1.1 | - |
| Whole milk | - | 5.6 | 3.7 |
| Skim milk | - | 2.9 | 1.5 |
| Gelatin | 1.4 | 1.2 | 0.6 |
| Enzymatically degraded gelatin | - | - | 1.1 |
| Wheat flour | - | 2.0 | 5.6 |
| Cheese | - | - | 3.2 |
| Carbohydrate material | | | |
| Refined sucrose | 3.0 | 1.7 | 1.7 |
| Fat materials | | | |
| Rice oil | 5.0 | 1.0 | 0.8 |
| Chocolate | - | - | - |
| Other ingredients | | | |
| Vitamins | q.s. | q.s. | q.s. |
| Minerals | q.s. | q.s. | q.s. |
| Flavors | q.s. | q.s. | q.s. |
| Viscosity, mPas (cp) (30°C) | 1240 | 2150 | 1880 |
| Amino acid score | 100 | 100 | 92 |

Table 1 (continued)

| Example No. | Example 7 | Example 8 | Example 9 |
|---|---|---|---|
| Protein (g/80 ml) | 12.0 | 8.8 | 10.0 |
| (w/w%) | 59.7 | 44.2 | 50.0 |
| Carbohydrate (g/80 ml) | 7.0 | 8.3 | 5.2 |
| (w/w%) | 34.8 | 41.7 | 26.0 |
| Fat (g/80 ml) | 1.1 | 2.8 | 4.8 |
| (w/w%) | 5.5 | 14.1 | 24.0 |
| Energy y (Kcal) | 0,360 (86) | 0,394 (94) | 0,435 (104) |
| Protein materials | | | |
| Casein | 6.9 | 4.9 | 5.0 |
| Sodium caseinate | 1.1 | 1.1 | 3.3 |
| Calcium caseinate | 2.2 | 1.7 | - |
| Whole milk | - | 1.5 | 5.6 |
| Skim milk | 1.5 | 0.9 | 1.5 |
| Gelatin | 0.9 | 0.6 | - |
| Enzymatically degraded gelatin | 1.1 | - | - |
| Wheat flour | 4.0 | 2.0 | - |
| Cheese | - | 3.8 | 3.2 |
| Carbohydrate material | | | |
| Refined sucrose | 3.3 | 5.7 | 0.5 |
| Fat materials | | | |
| Rice oil | 0.9 | 1.0 | 1.0 |
| Chocolate | - | - | 3.0 |
| Other ingredients | | | |
| Vitamins | q.s. | q.s. | q.s. |
| Minerals | q.s. | q.s. | q.s. |
| Flavors | q.s. | q.s. | q.s. |
| Viscosity, mPas (cp) (30°C) | 2310 | 1250 | 1220 |
| Amino acid score | 100 | 100 | 100 |

Table 1 (continued)

| Example No. | Example 10 | Example 11 | Example 12 | Example 13 |
|---|---|---|---|---|
| Protein (g/80 ml) | 9.6 | 9.8 | 11.2 | 9.4 |
| (w/w%) | 48.7 | 51.0 | 52.1 | 49.5 |
| Carbohydrate (g/80 ml) | 6.7 | 6.3 | 7.1 | 6.3 |
| (w/w%) | 34.0 | 32.8 | 33.0 | 33.2 |
| Fat (g/80 ml) | 3.4 | 3.1 | 3.2 | 3.3 |
| (w/w%) | 17.3 | 16.1 | 14.9 | 17.4 |
| y | 0,402 | 0,385 | 0,427 | 0,389 |
| Energy (Kcal) | (96) | (92) | (102) | (93) |
| Protein materials | | | | |
| Casein | 4.9 | 5.6 | 6.3 | 4.8 |
| Sodium caseinate | 3.1 | - | - | 1.5 |
| Calcium caseinate | - | 2.9 | 3.0 | 1.8 |
| Whole milk | 3.5 | 4.3 | 2.5 | 3.2 |
| Skim milk | 1.8 | - | 3.8 | 2.5 |
| Gelatin | 0.4 | 0.7 | 0.2 | - |
| Enzymatically degraded gelatin | - | - | 0.4 | - |
| Wheat flour | 1.0 | 1.4 | 0.8 | 1.0 |
| Cheese | 3.6 | 2.4 | - | 1.4 |
| Carbohydrate material | | | | |
| Refined sucrose | 3.5 | 3.5 | 1.7 | 1.5 |
| Fat materials | | | | |
| Rice oil | 1.2 | 1.1 | 1.1 | 0.9 |
| Chocolate | - | - | 3.3 | 2.6 |
| Other ingredients | | | | |
| Vitamins | q.s. | q.s. | q.s. | q.s. |
| Minerals | q.s. | q.s. | q.s. | q.s. |
| Flavors | q.s. | q.s. | q.s. | q.s. |
| Viscosity, mPas (cp) (30°C) | 760 | 900 | 2500 | 1000 |
| Amino acid score | 100 | 100 | 100 | 100 |

The food compositions thus obtained in the above Examples were not only effective in augmenting body protein but also suited as a tube feeding system for patients with adult disease. Actually the use of the compositions were all effective in preventing exacerbation of adult disease or preventing onset of the disease.

When the foregoing examples were repeated except that erythritol and/or lactosucrose was used as the carbohydrate in lieu of part or the whole of refined sucrose, all the resulting compositions were also able to produce the nutritional supplementation effect of the invention.

## Claims

1. A high-protein, high-viscosity nutritive food composition comprising, on a dry weight basis, 40 to 65 weight % of protein, 5 to 25 weight % of fat and 15 to 40 weight % of carbohydrate and having a viscosity of 500 to 3.000 mPas (500 to 3,000 cp) (at 30°C, Type-B viscosimeter) and an amino acid score (on a 2~5-year-old infant basis) of not less than 80.

2. The high-protein, high-viscosity nutritive food composition of claim 1 wherein the protein accounts for 40 to 53 weight %.

3. The high-protein, high-viscosity nutritive food composition of claim 1 wherein the fat accounts for 10 to 18 weight %.

4. The high-protein, high-viscosity nutritive food composition of claim 1 wherein the carbohydrate accounts for 20 to 35 weight %.

5. The high-protein, high-viscosity nutritive food composition of claim 1 wherein the protein accounts for 40-53 weight % and the fat accounts for 10 to 18 weight %.

6. The high-protein, high-viscosity nutritive food composition of claim 1 wherein the fat accounts for 10 to 18 weight % and the carbohydrate accounts for 20 to 35 weight %.

7. The high-protein, high-viscosity nutritive food composition of claim 1 wherein the protein accounts for 40 to 53 weight % and the carbohydrate accounts for 20 to 35 weight %.

8. The high-protein, high-viscosity nutritive food composition of claim 1 wherein the protein accounts for 40 to 53 weight %, the fat accounts for 10 to 18 weight % and the carbohydrate accounts for 20 to 35 weight %.

## Patentansprüche

1. Nahrhafte, proteinreiche Lebensmittelzusammensetzung hoher Viskosität, die, auf Trockengewichtsbasis, 40 bis 65 Gew.% Protein, 5 bis 25 Gew.% Fett und 15 bis 40 Gew.% Kohlenhydrate enthält, eine Viskosität von 500 bis 3000 mPas (500 bis 3000 cp) (bei 30°C, Viskosimeter Typ B) aufweist und einen Aminosäure-Score (bezogen auf ein 2 bis 5 Jahre altes Kleinkind) von nicht weniger als 80 hat.

2. Nahrhafte proteinreiche Lebensmittelzusammensetzung hoher Viskosität nach Anspruch 1, in der das Protein 40 bis 53 Gew.% ausmacht.

3. Nahrhafte, proteinreiche Lebensmittelzusammensetzung hoher Viskosität nach Anspruch 1, in der das Fett 10 bis 18 Gew.% ausmacht.

4. Nahrhafte, proteinreiche Lebensmittelzusammensetzung hoher Viskosität nach Anspruch 1, in der die Kohlenhydrate 20 bis 35 Gew.% ausmachen.

5. Nahrhafte, proteinreiche Lebensmittelzusammensetzung hoher Viskosität nach Anspruch 1, in der das Protein 40 bis 53 Gew.% und das Fett 10 bis 18 Gew.% ausmacht.

6. Nahrhafte, proteinreiche Lebensmittelzusammensetzung hoher Viskosität nach Anspruch 1, in der das Fett 10 bis 18 Gew.% ausmacht und die Kohlenhydrate 20 bis 35 Gew.% ausmachen.

7. Nahrhafte, proteinreiche Lebensmittelzusammensetzung hoher Viskosität nach Anspruch 1, in der das Protein 40 bis 53 Gew.% ausmacht und die Kohlenhydrate 20 bis 35 Gew.% ausmachen.

8. Nahrhafte, proteinreiche Lebensmittelzusammensetzung hoher Viskosität nach Anspruch 1, in der das Protein 40 bis 53 Gew.% ausmacht, das Fett 10 bis 18 Gew.% ausmacht und die Kohlenhydrate 20 bis 35 Gew.% ausmachen.

## Revendications

1. Composition alimentaire nutritive à haute teneur en protéines et de viscosité élevée, comprenant, sur une base en poids sec, 40 à 65% en poids de protéines, 5 à 25% en poids de graisses et 15 à 40% en poids d'hydrates de carbone et ayant une viscosité de 500 à 3.000 mPa.s (500 à 3.000 cp) (viscosimètre de type B, à 30°C) et une note d'acides aminés (sur la base d'un enfant de 2 à 5 ans) d'au moins 80.

2. Composition alimentaire nutritive à haute teneur en protéines et de viscosité élevée suivant la revendication 1, dans laquelle les protéines sont présentes en une quantité de 40 à 53% en poids.

3. Composition alimentaire nutritive à haute teneur en protéines et de viscosité élevée suivant la revendication 1, dans laquelle les graisses sont présentes en une quantité de 10 à 18% en poids.

4. Composition alimentaire nutritive à haute teneur en protéines et de viscosité élevée suivant la revendication 1, dans laquelle les hydrates de carbone sont présents en une quantité de 20 à 35% en poids.

5. Composition alimentaire nutritive à haute teneur en protéines et de viscosité élevée suivant la revendication 1, dans laquelle les protéines sont présentes en une quantité de 40 à 53% en poids et les graisses sont présentes en une quantité de 10 à 18% en poids.

6. Composition alimentaire nutritive à haute teneur en protéines et de viscosité élevée suivant la revendication 1, dans laquelle les graisses sont présentes en une quantité de 10 à 18% en poids et les hydrates de carbone sont présents en une quantité de 20 à 35% en poids.

7. Composition alimentaire nutritive à haute teneur en protéines et de viscosité élevée suivant la revendication 1, dans laquelle les protéines sont présentes en une quantité de 40 à 53% en poids et les hydrates de carbone sont présents en une quantité de 20 à 35% en poids.

8. Composition alimentaire nutritive à haute teneur en protéines et de viscosité élevée suivant la revendication 1, dans laquelle les protéines sont présentes en une quantité de 40 à 53% en poids, les graisses sont présentes en une quantité de 10 à 18% en poids et les hydrates de carbone sont présents en une quantité de 20 à 35% en poids.